# EUROPÄISCHE PATENTSCHRIFT

(11) **EP 0 768 882 B1**
(45) Veröffentlichungstag und Bekanntmachung des Hinweises auf die Patenterteilung: **19.08.1998**
(21) Anmeldenummer: 95922407.2
(22) Anmeldetag: 22.06.1995
(51) Int. Cl.: A61K 31/55, A61K 9/28

(54) **CARBAMAZEPIN-ARZNEIFORM MIT VERZÖGERTER WIRKSTOFFFREISETZUNG**
CARBAMAZEPINE MEDICAMENT WITH RETARDED ACTIVE SUBSTANCE RELEASE
MEDICAMENT A LA CARBAMAZEPINE A LIBERATION RETARDEE DU PRINCIPE ACTIF

(30) Priorität: 01.07.1994 DE 4423078
(43) Veröffentlichungstag der Anmeldung: 23.04.1997
(73) Patentinhaber: ARZNEIMITTELWERK DRESDEN GmbH, D-01435 Radebeul (DE)
(72) Erfinder: LANDGRAF, Karl-Friedrich, 01217 Dresden (DE); REISS, Sabine, 01445 Radebeul (DE); SCHUBERT, Eberhard, 01445 Radebeul (DE)
(86) Internationale Anmeldenummer: DE9500805
(87) Internationale Veröffentlichungsnummer: WO9601112

(56) Entgegenhaltungen:
- EP-A- 0 080 341
- EP-A- 0 388 954
- DE-A- 3 725 824
- GB-A- 2 122 490
- Embase Abstract Nr. 93329228 & Pharm. Ind. 1993, Band 55, Nr. 10, S. 940-947 in der Anmeldung erwähnt
- DRUG DEVELOPMENT AND INDUSTRIAL PHARMACY, Bd. 17, Nr. 13, 1991 Seiten 1753-1764, P.GUINCHEDI ET AL. 'Carbamazepine modified release dosage form'

## Beschreibung

Die Erfindung betrifft ein Verfahren zur Herstellung einer oral applizierbaren Carbamazepin-Arzneiform mit verzögerter Wirkstofffreisetzung.
Carbamazepin, ein 5 H-Dibenz[*b,f*]azepin-5-carboxamid, wird insbesondere als Antiepileptikum eingesetzt.
Handelsübliche Darreichungsformen sind Tabletten mit 200 mg Wirkstoff, Retardtabletten mit 200 bis 600 mg Wirkstoff und Sirupe.

Bekannterweise bildet Carbamazepin bei Kontakt mit Wasser in kürzester Zeit ein Dihydrat. Schon beim Granulieren im Intensivmischer oder im Wirbelschichtgranulator mit wäßrigen Granulierflüssigkeiten setzt die Dihydratbildung ein. Dieses Dihydrat liegt kristallin in Form von Nadeln vor, welche bis zu einer Teilchengröße von 500 µm anwachsen können. Das wirkt sich negativ auf die Weiterverarbeitung, besonders bei der Ausbildung von retardierenden Überzügen aus.
Die bekannten Herstellungsverfahren verzichten deshalb auf wäßrige Medien und bevorzugen den Einsatz von organischen Lösungsmitteln.
Die Retardierung eines Wirkstoffs kann auf unterschiedliche Art und Weise erfolgen. Für Carbamazepins wird in der DE-PS 32 77 520 eine Formulierung beschrieben, bei der der Wirkstoff mit üblichen Tablettierhilfsstoffen gemischt und zu einem Kern verpreßt oder in Kapseln gefüllt wird. Der Kern bzw. die Kapsel wird mit einem in Isopropanol gelösten Methacrylsäure-Methacrylsäuremethylester-Gemisch, das Acetyltributylcitrat als Weichmacher enthält, überzogen. Durch den Einsatz eines organischen Lösungsmittels wird so die Dihydratbildung des Carbamazepins verhindert.
In der DE-PS 38 68 077 und DE-PS 37 25 824 wird eine Carbamazepin-Hilfsstoffkomposition beansprucht, die ein Schutzkolloid enthält, welches das Kristallwachstum von Carbamazepin in Gegenwart von Wasser inhibiert.
Der carbamazepinhaltige Kern wird hier mit einer organischen Lösung von Celluloseacetat überzogen. In den Film wird in geeigneter Weise eine Passage in Form eines Loches eingebracht.

Die Verfahren zur Herstellung dieser Formulierungen haben den Nachteil, daß die Arbeiten mit organischen Lösungsmitteln ausgeführt werden müssen, was eine Umweltbelastung zur Folge hat beziehungsweise hohen Aufwand und Kosten bedeutet.
Weiterhin sind diese wie oben beschrieben hergestellten Arzneiformen (Tabletten bzw. Kapseln) nicht teilbar, da bei einer Teilung die Hülle beschädigt wird und damit auch die Retardwirkung verloren geht.
Somit sind die Dosierungsmöglichkeiten einschränkt.

Desweiteren sind Zubereitungen (Tabletten) bekannt, die bei einer Teilung oder bei dem Zerfall in einzelne Partikel in Flüssigkeiten außerhalb oder innerhalb des Magen-Darm-Trakts ihre Retardwirkung nicht verlieren.
In Pharm. Ind. 55, Nr.10 (1993) S. 940-947 werden feste orale Zubereitungen , in denen einzelne Partikel mit wäßrigen Dispersionen aus Copolymeren der Methacrylsäure und Methylmethacrylsäureestern überzogen und zu Tabletten verpreßt werden, beschrieben. Durch Zugabe von 25-50 % Hilfsstoffe wird ein schnellerer Zerfall der Tabletten erreicht. Die Zugabe eines Weichmachers ermöglicht eine wesentliche Erhöhung der Reißdehnung des Überzuges und gewährleistet die mechanische Stabilität.
Genannt wird hierbei der Überzug von Paracetamol-, Kaliumchlorid- und Acetylsalicylsäure-Wirkstoffkristallen, Theophyllin-Granulat und Indomedacin- und Theophyllin-Pellets.

Der vorliegenden Erfindung liegt die Aufgabe zugrunde, ein geeignetes Verfahren zur Herstellung einer Carbamazepin-Arzneiform bereitzustellen, in welchem trotz Einsatz von Wasser als Lösungs- oder Dispersionsmittel das mit der Dihydratbildung des Carbamazepins verbundene Kristallwachstum verhindert und damit die Freisetzung des Carbamazepins ausreichend verzögert wird.

Die Aufgabe wurde erfindungsgemäß dadurch gelöst, daß Filmbildner in Kombination mit einem Weichmacher als wäßrige Lösung und/oder Dispersion auf Carbamazepin aufgesprüht werden. Das überzogene Carbamazepin kann gegebenenfalls mit weiteren Hilfsstoffen gemischt und zu Tabletten verpreßt oder in Kapseln gefüllt werden.

Für die Retardierung des Carbamazepin werden als Filmbildner Polymethacrylatdispersionen eingesetzt. Bevorzugt werden dabei:
- Gemisch aus Polyethylacrylat und Polymethylmethacrylat im Verhältnis 2 : 1 (Eudragit® NE 30D),
- Gemisch aus Polyethylacrylat, Polymethylmethacrylat und Polytrimethylammonioethylmethacrylat-chlorid im Gemisch 1 : 2 : 0,1 (Eudragit® RS 30 D) oder
- das vorstehend genannte Gemisch im Verhältnis 1 : 2 : 0,2 (Eudragit® RL 30 D)

Als wasserlösliche Weichmacher kommen beispielsweise Glyceroltriacetat oder Triethylcitrat in Frage. Diese werden in einem Verhältnis von Lacktrockensubstanz zu Weichmacher wie 1 : 0,05 bis 1 : 0,25, vorzugsweise von 1 : 0,15 bis 1 : 0,22 eingesetzt.

Das Verhältnis von Carbamazepin zu Filmbildner zu richtet sich nach dem zu erzielenden Retardierungseffekt und beträgt 1 : 0,03 bis 1 : 0,5.
Insbesondere wird ein Verhältnis von Carbamazepin zu Filmbildner von 1 : 0,05 bis 1 : 0,1 angewendet, vorzugsweise jedoch 1 : 0,05 bis 1 : 0,08.

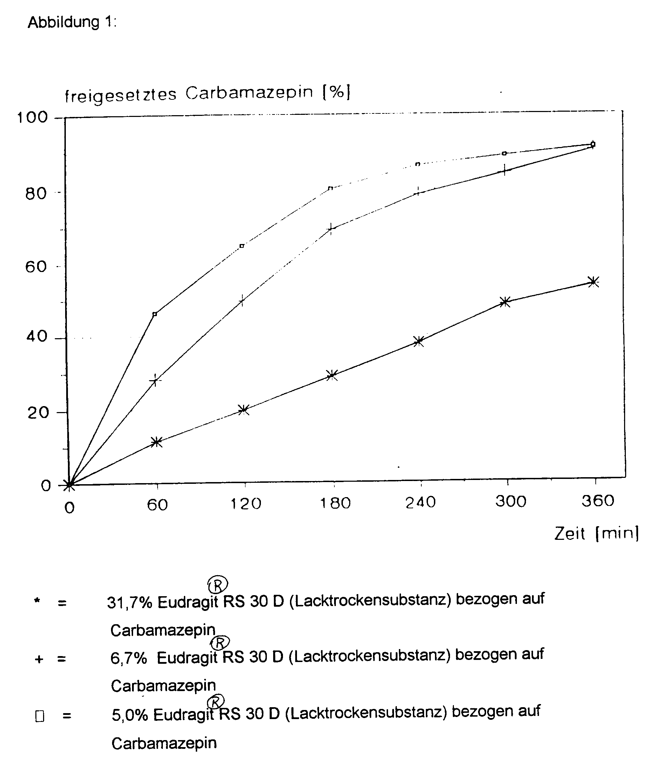

Die Freisetzungskurven für solche unterschiedlichen Verhältnisse von Carbamazepin zu Filmbildner sind in der Abb. 1 dargestellt. Als Methode wurde der Dissolutins Test der USP XXII für Carbamazepin angewendet (Medium: Wasser mit 1 % Natriumdodecylsulfatzusatz).

Überraschenderweise konnte durch die erfindungsgemäße Zusammensetzung der Carbamazepin-Arzneiform die bekanntermaßen bei Kontakt des Carbamazepins mit Wasser spontan einsetzende Dihydratbildung, die verbunden ist mit einem nadelförmigen Kristallwachstum und damit schlechten Verarbeitbarkeit verhindert werden.

Der Filmbildner in Kombination mit einem Weichmacher wird als wäßrige Lösung und/oder Dispersion vorteilhafterweise in einem Wirbelschichtgranulator aufgesprüht.
Zur Dispersion können zusätzlich zur Verhinderung des Verklebens der überzogenen Partikel Trennmittel zugesetzt und/oder in der Wirbelschicht als als separate Suspension nachträglich aufgesprüht werden.
Beispielsweise wird Talkum in einem Konzentrationsverhältnis von Lacktrockensubstanz zu Trennmittel wie 1 : 0,4 bis 1 : 1, vorzugsweise 1 : 0,45 bis 1 : 0,55 aufgetragen.

Den überzogenen Carbamazepinkristallen können in an sich bekannter Weise weitere galenische Hilfsstoffe zugemischt werden. Die so hergestellten Mischungen oder auch die überzogenen Kristalle lassen sich dann in Hartgelatinekapseln abfüllen oder zu teilbaren Tabletten verpressen. Die mit der genannten Weiterverarbeitung der überzogenen Carbamazepins verbundene mechanische Belastung der Einzelpartikel, insbesondere bei der Tablettierung, führt nicht zur Beschädigung des Filmüberzugs.

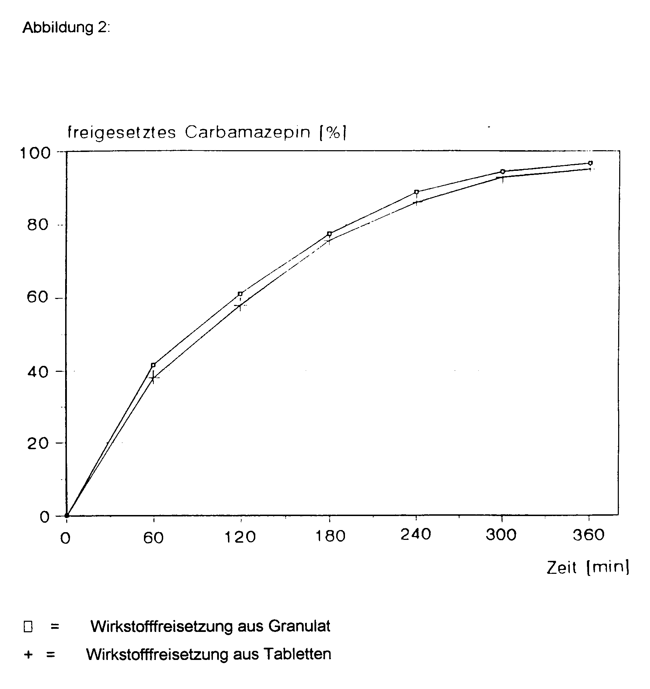

Die Endarzneiform zeigt die gleiche Verzögerung der Lösungsgeschwindigkeit, geprüft nach der Methode der USP XXII für Carbamazepin, wie das überzogene Carbamazepin (Abb.2).

Das erfindungsgemäße Verfahren soll anhand der Ausführungsbeispiele näher erläutert werden:

### Beispiel 1

Aus 2,23 kg Eudragit® RS 30 D, 135 g Glyceroltriacetat gelöst in 2,35 l Wasser und 325 g Talkum suspendiert in 1 l Wasser wird eine Suspension hergestellt.
Die Suspension wird im Wirbelschichtgranulator WSG 15 (Fa.Glatt) auf 10 kg Carbamazepin augesprüht. Danach wird eine Suspension von 625 g Talkum in 2 l Wasser aufgesprüht. Das so erhaltene Granulat wird mit 914 g Mikrokristalliner Cellulose, 653 g unlöslichen Polyvidon, 70 g hochdispersen Siliciumdioxid und 35 g Magnesiumstearat gemischt.
Die Mischung wird zu Tabletten mit einem Wirkstoffgehalt von 200, 400 oder 600 mg Carbamazepinn verpreßt oder die entsprechende Menge Granulat wird in Kapseln der Größe 1 gefüllt.

### Beispiel 2

Aus 340 g Eudragit® RS 30 D, 20,4 g Triethylcitrat gelöst in 0,3 l Wasser und 40 g Talcum suspendiert in 0,1 l Wasser wird eine Suspension hergestellt. Die Suspension wird im Wirbelschichtgranulator GPCG 1 (Fa.Glatt) bei einer Produkttemperatur von 27 - 30°C auf 1 kg Carbamazepin aufgesprüht.
Das so erhaltene Granulat wird mit 65 g Mikrokristalliner Cellulose, 65 g unlöslichen Polyvidon, 7 g hochdispersen Siliciumdioxid und 3,5 g Magnesiumstearat gemischt. Die Mischung wird zu Tabletten mit einem Wirkstoffgehalt von 200, 400 oder 600 mg Carbamazepinn verpreßt oder die entsprechende Menge Granulat wird in Kapseln der Größe 1 gefüllt.

### Beispiel 3

Aus 29 g Eudragit® NE 30 D, 1,3 g Glyceroltriacetat gelöst in 0,03 l Wasser und 9 g Talcum suspendiert in 0,03 l Wasser wird eine Suspension hergestellt. Die Suspension wird im Wirbelschichtgranulator UNI Glatt bei einer Produkttemperatur von 27 - 30°C auf 250 g Carbamazepin aufgesprüht. Das so erhaltene Granulat wird mit 16 g Mikrokristalliner Cellulose, 16 g unlöslichen Polyvidon, 2 g hochdispersen Siliciumdioxid und 1 g Magnesiumstearat gemischt. Die Mischung wird zu Tabletten mit einem Wirkstoffgehalt von 200, 400 oder 600 mg Carbamazepinn verpreßt oder die entsprechende Menge Granulat wird in Kapseln der Größe 1 gefüllt.

## Patentansprüche

1. Verfahren zur Herstellung einer Carbamazepin - Arzneiform mit verzögerter Wirkstofffreisetzung dadurch gekennzeichnet, daß Filmbildner in Kombination mit Weichmacher als wäßrige Lösung und/oder Dispersion auf Carbamazepin in einem Wirbelschichtgranulator aufgesprüht werden.

2. Verfahren nach Anspruch 1 dadurch gekennzeichnet, daß als Filmbildner Polymethacrylatgemische wie Polyethylacrylat und Polymethylmethacrylat im Verhältnis 2 : 1, Polyethylacrylat, Polymethyl-methacrylat und Polytrimethylammonioethylmethacrylat-chlorid im Gemisch 1 : 2 : 0,1 oder 1 : 2 : 0,2 eingesetzt werden.

3. Verfahren nach Anspruch 1 dadurch gekennzeichnet, daß das Verhältnis von Carbamazepin zu Filmbildner 1 : 0,03 bis 1 : 0,5 beträgt.

4. Verfahren nach Anspruch 1 dadurch gekennzeichnet, daß Filmbildner in Kombination mit Weichmacher im Verhältnis 1 : 0,05 bis 1 : 0,25 eingesetzt werden.

5. Verfahren nach den Ansprüchen 1 und 4 dadurch gekennzeichnet, daß das Verhältnis Filmbildner zu Weichmacher vorzugsweise 1 : 0,15 bis 1 : 0,22 beträgt.

6. Verfahren nach den Ansprüchen 1 , 4 und 5 dadurch gekennzeichnet, daß als Weichmacher Glyceroltriacetat oder Triethylcitrat eingesetzt werden.

7. Verfahren nach Anspruch 1 dadurch gekennzeichnet, daß gegebenenfalls der Filmbildnerdispersion Trennmittel, wie Talkum, im Verhältnis 1 : 0,4 bis 1 1 zugesetzt und/oder in der Wirbelschicht als separate Suspension nachträglich aufgesprüht werden.

8. Verfahren nach den Ansprüchen 1 bis7 dadurch gekennzeichnet, daß das überzogene Carbamazepin mit weiteren Hilfsstoffen gemischt, zu teilbaren Tabletten verpreßt oder in Kapseln gefüllt wird.

9. Verfahren nach den Ansprüchen 1 bis 7 dadurch gekennzeichnet, daß das überzogenen Carbamazepin ohne weitere Hilfsstoffe in Kapseln gefüllt wird.

## Claims

1. Process for the production of a carbamazepine pharmaceutical form with delayed release of active compound, characterized in that film-forming agents in combination with plasticizers are sprayed as an aqueous solution and/or dispersion onto carbamazepine in a fluidized bed granulator.

2. Process according to Claim 1, characterized in that the film-forming agents employed are polymethacrylate mixtures such as poly(ethyl acrylate) and poly(methyl methacrylate) in the ratio 2:1, poly(ethyl acrylate), poly(methyl methacrylate) and poly(trimethylammonioethyl methacrylate chloride) in a 1:2:0.1 or 1:2:0.2 mixture.

3. Process according to Claim 1, characterized in that the ratio of carbamacepine to film-forming agent is 1:0.03 to 1:0.5.

4. Process according to Claim 1, characterized in that film-forming agents in combination with plasticizers are employed in the ratio 1:0.05 to 1:0.25.

5. Process according Claims 1 and 4, characterized in that the ratio of film-forming agent to plasticizer is preferably 1:0.15 to 1:0.22.

6. Process according to Claims 1, 4 and 5, characterized in that the plasticizer employed is glycerol triacetate or triethyl citrate.

7. Process according to Claim 1, characterized in that, if appropriate, release agents, such as talc, are added to the film-forming agent dispersion in the ratio 1:0.4 to 1:1 and/or subsequently sprayed on in the fluidized bed as a separate suspension.

8. Process according to Claims 1 to 7, characterized in that the coated carbamazepine is mixed with further auxiliaries, compressed to give divisible tablets or dispensed into capsules.

9. Process according to Claims 1 to 7, characterized in that the coated carbamazepine is dispensed into capsules without further auxiliaries.

## Revendications

1. Procédé de préparation d'une forme de médicament à la carbamazépine présentant une libération retardée de la substance active, caractérisé en ce que 1'on pulvérise, dans un granulateur à lit fluidisé, des agents filmogènes en combinaison avec un plastifiant sous forme de solution et/ou dispersion aqueuse sur de la carbamazépine.

2. Procédé selon la revendication 1, caractérisé en ce que 1'on utilise en tant qu'agents filmogènes, des mélanges à base de polyméthacrylate, tels que du poly(acrylate d'éthyle) et du poly(méthacrylate de méthyle), en un rapport de 2:1, du poly(acrylate d'éthyle, du poly(méthacrylate de méthyle) et du chlorure de poly(méthacrylate de triméthyle ammonioéthyle) en un mélange à 1:2:0,1 ou 1:2:0,2.

3. Procédé selon la revendication 1, caractérisé en ce que le rapport entre carbamazépine et agent filmogène est de 1:0,03 à 1:0,5.

4. Procédé selon la revendication 1, caractérisé en ce que 1'on utilise, des agents filmogènes en combinaison avec un plastifiant en un rapport de 1:0,05 à 1:0,25.

5. Procédé selon les revendications 1 et 4, caractérisé en ce que le rapport entre agent filmogène et plastifiant est de préférence de 1:0,15 à 1:0,22.

6. Procédé selon les revendications 1, 4 et 5, caractérisé en ce que 1'on utilise en tant que plastifiant, le triacétate de glycérol ou le citrate de triéthyle.

7. Procédé selon la revendication 1, caractérisé en ce que, le cas échéant, on ajoute à la dispersion d'agent filmogène, des agents de séparation, tels que du talc, en un rapport de 1:0,4 à 1:1 et/ou on les pulvérise ultérieurement dans le lit fluidisé sous forme d'une suspension séparée.

8. Procédé selon les revendications 1 à 7, caractérisé en ce que la carbamazépine enrobée est mélangée avec des auxiliaires supplémentaires, pressée en comprimés divisibles ou chargée dans des capsules.

9. Procédé selon les revendications 1 à 7, caractérisé en ce que la carbamazépine enrobée est chargée dans des gélules sans auxiliaire supplémentaire.
